# EUROPEAN PATENT SPECIFICATION

(11) **EP 1 814 511 B1**
(45) Date of publication and mention of the grant of the patent: **23.12.2009**
(21) Application number: 05808282.7
(22) Date of filing: 17.11.2005
(51) Int. Cl.: A61K 8/46, A61K 8/73, A61Q 5/04

(54) **METHOD FOR THE PERMANENT SHAPING OF HAIR USING A CATIONIC CELLULOSE DERIVATIVE**
VERFAHREN ZUR PERMANENTEN FORMUNG VON HAAR MIT EINEM KATIONISCHEN ZELLULOSEDERIVAT
PROCEDE DESTINE AU PERMANENTAGE DES CHEVEUX AU MOYEN D'UN DERIVE CELLULOSIQUE CATIONIQUE

(30) Priority: 24.11.2004 DE 102004056801; 27.01.2005 DE 102005003748
(43) Date of publication of application: 08.08.2007
(73) Proprietor: The Procter and Gamble Company, Cincinnati, Ohio 45202 (US)
(72) Inventor: CASSIER, Thorsten, 64807 Dieburg (DE); SCHREIBER, Birgit, 64678 Lindenfels (DE)
(74) Representative: Hirsch, Uwe Thomas M.H.
(86) International application number: PCT/EP2005/012309
(87) International publication number: WO 2006/056361

(56) References cited:
- EP-A- 0 686 388
- WO-A-01/48021
- WO-A-99/02122
- WO-A-2005/000903
- US-A- 4 663 159

## Description

The present invention involves a thickened agent to carry out the reductive treatment in permanent hair shaping, wherein the agent contains a certain cationic cellulose derivative, as well as a method for the permanent shaping of hair using this agent.

In permanent hair shaping, hair is normally first treated with a shaping agent based on a keratin-reducing mercapto compound, which causes an opening of the disulfide bridges of the hair keratin, after which the hair is set the desired shape. Keratin-reducing mercapto compounds, such as salts or esters of mercaptocarboxylic acids, are normally used as the shaping agent. The hair is then rinsed with water and given an oxidative post-treatment with a fixing agent. In this process, the previously split disulfide bridges are re-linked in the new shape.

The chemical and mechanical processes that act on the hair with this procedure worsen hair quality, particularly with respect to hold and combability of the hair, and the hair becomes brittle and rough.

There have already been attempts to give hair a smooth hold as well as to improve the combability of the hair by adding certain conditioning substances to the shaping agent. The use of cationic compounds, for example, cationic polymers, has proven to be particularly advantageous in this process.

However, all of the hair shaping agents described have disadvantages. For example, due to insufficient thickening, these agents can cause excess product to come into contact with the scalp, and thus cause unpleasant scalp irritations, or if the product is too thick, this can lead to an unsatisfactory shaping of the hair.

Some thickeners, however, do not provide any cosmetic appearance to the shaping agent, because they are inhomogeneous or have a slimy, stringy, or pudding-like consistency.

In addition, long-lasting thickeners based on polymers are not stable over a period of several months in the presence of reducing agents and with regard to viscosity (phase separation, precipitate) and high pH values,or they impart a red coloring with the wave agent thioglycolatedue to their heavy metal content. A particular problem presents itself if the product is too runny, which causes the wave agent to come into contact with the scalp.

The use of certain cationic cellulose derivatives in agents for conditioning the skin and hair, such as shampoos and hair conditioning agents, is described in WO 2005/000903 A1 - however not for agents that are aggressive towards hair, and above all those that contain strong reducing agents or oxidizing agents.

Although numerous cationic cellulose derivatives in pH-neutral or mildly acidic hair conditioning agents have already been recommended, there are only a few that are sufficiently stable to permit their use in strongly alkaline, reducing hair shaping agents.

There is a challenge to provide an agent for carrying out the reductive step in permanent hair shaping that does not drain from the hair during the treatment period, is not runny, but that nevertheless wets the hair well, can be present as a clear gel, is able to condition the hair well, can be rinsed out readily with water, where this agent imparts a good cosmetic appearance, is stable and at the same time ensures good, uniform shaping results from the hairline to the hair tips.

Unexpectedly, it was found that the stated challenge could be overcome in an outstanding fashion when the hair shaping agent used to carry out the reductive step in a permanent hair shaping contains a cationic cellulose derivative, which is substituted with_ substituents of Formulas (I) and (II).

The object of the present invention is thus an agent as recited in Claim 1 as well as a method for permanent hair shaping as recited in Claim 21.

The preferred embodiments of the invention are elaborated in the subclaims.

The advantages of the hair shaping agent, in addition to simpler application as a result of the increase in viscosity, include in particular a significant improvement in the hold and combability of the permanently restructured hair, as well as especially uniform shaping results from the hairline to the hair tips.

Cationic polymers of Formula (III) are preferred where X represents a number from 0.1 to 0.6, Y is a number from 0.1 to 0.6, U and V each represent a number from 0.001 to 0.06, R represents a straight-chain alkyl residue of from 8 to 18 carbon atoms, and n is an integer from 1,000 to 6,000.

Cationic polymers of Formula (III) are especially preferred when X = 0.15 to 0.35, Y = 0.15 to 0.35, U and V each represent a number from 0.001 to 0.06, R = a straight-chain alkyl residue of from 10 to 16 carbon atoms and n stands for 2,000 to 6,000. Especially preferred is R = a straight-chain alkyl residue with 12 carbon atoms.

Also preferred are cationic polymers of Formula (IV).

It is advantageous for the hair shaping agent to contain a cationic cellulose derivative as recited in Claim 1 in an amount of from 0.01 to 5 percent by weight, preferably in an amount of from 0.1 to 2 percent by weight and especially preferably in an amount of from 0.1 to 0.5 percent by weight.

Cationic cellulose derivatives as recited in Claim 1 are sold commercially by the DOW CHEMICAL/Amerchol Company under the names Polymer SL^{®}-5, Polymer SL^{®}-30, Polymer SL^{®}-60, and Polymer SL^{®}-100, wherein the degree of hydrophobization (due to the increasing chain length of the R⁴ substituent or to an increased value for the degree of substitution for the substituent (I)), increases with as the number grows from 5 to 30, 60 and 100. Their manufacture is described for example in WO 2005/000903 A1. In particular, cellulose ethers used in the manufacture of the cellulose derivatives include hydroxyethylcellulose, hydroxypropylcellulose, hydroxyethylmethylcellulose, hydroxypropylmethylcellulose and methylcellulose, among which hydroxyethylcellulose and hydroxypropylcellulose are preferred.

The cationic cellulose derivatives as recited in Claim 1 have a molecular weight of 10,000 to 4,000,000, preferably from 100,000 to 1,000,000, and the viscosity of a 1% aqueous solution is preferably from 1,000 to 5,000 mPa · sec at 25 degrees Celsius (77 °F), and especially preferably from 1,500 to 3,000 mPa · sec at 25 degrees Celsius (77 °F). The viscosity values are based on measurements with a Haake rotational viscometer, type VT 550, with a shear rate of 10.0 per second. A model NV or MV double-gap cylinder measuring system was used.

Unexpectedly, it was also observed that a cationic cellulose derivative as recited in Claim 1 as a component of the agent of the present invention, acts in a synergistic fashion in combination with an additional cationic conditioning polymer to provide an enhancement of the conditioning properties, especially the hold and the combability of the hair, under both dry and moist conditions. In addition, a further improved result was observed with respect to the uniformity of the hair shaping from the hairline to the tips.

Hair-conditioning polymers that can be used to act synergistically in combination with the cationic cellulose derivatives as recited in Claim 1 can be homo- or copolymers, where quaternary nitrogen groups are contained either in the polymer chain or preferably as substituents on one or more of the monomers. The ammonium group-containing monomers can be copolymerized with non-cationic monomers. Suitable cationic monomers are unsaturated, radical-polymerizable compounds, which bear at least one cationic group, especially ammonium-substituted vinyl monomers such as for example trialkylmethacryloxyalkylammonium, trialkylacryloxyalkylammonium, dialkyldiallylammonium and quaternary vinylammonium monomers with cyclic, cationic nitrogen-containing groups such as pyridinium, imidazolium or quaternary pyrrolidones, for example alkyl vinyl imidazolium, alkyl vinyl pyridinium, or alkyl vinyl pyrrolidone salts. The alkyl groups of these monomers are preferably lower alkyl groups such as for example C-₁ to C-₇ alkyl groups, and especially preferably C-₁ to C-₃ alkyl groups.

Suitable noncationic comonomers are for example acrylamide, methacrylamide, alkyl- and dialkylacrylamide, alkyl- and dialkylmethacrylamide, alkyl acrylate, alkyl methacrylate, vinyl caprolactone, vinyl caprolactam, vinylpyrrolidone, vinyl esters such as vinyl acetate, vinyl alcohol, propylene glycol or ethylene glycol, where the alkyl groups in these monomers are preferably C-₁ to C-₇ alkyl groups, and especially preferably C-₁ to C-₃ alkyl groups.

Suitable polymers with quaternary ammonium groups are for example the polymers described under the name of POLYQUATERNIUM in the "CTFA Cosmetic Ingredient Dictionary", such as for example methylvinylimidazolium chloride/vinylpyrrolidone copolymer (POLYQUATERNIUM-16) or quaternized vinylpyrrolidone/dimethylaminoethyl methacrylate copolymer (POLYQUATERNIUM-11). Homo- and copolymers from dimethyldiallylammonium chloride, such as for example polydimethyldiallylammonium chloride (POLYQUATERNIUM-6), sold commercially by the Ondeo Nalco Company under the trade name Merquat^{®} 100, or the copolymers from acrylamide and dimethyldiallylammonium chloride (CTFA: POLYQUATERNIUM-7); quaternized hydroxyethyl cellulose (POLYQUATERNIUM-10) or quaternized guar derivatives.

Among the cationic polymers that can be contained in the agent of the present invention, the following examples are suitable: vinyl pyrrolidone/dimethylaminoethyl methacrylate methosulfate copolymer, sold under the trade names Gafquat^{®} 755 N and Gafquat^{®} 734 by Gaf Co. in the USA; Gafquat^{®} 734 is especially preferred. Other cationic polymers are, for example, the copolymer sold by BASF in Germany under the trade names LUVIQUAT^{®} HM 550 and LUVIQUAT^{®} HM 552, made of polyvinylpyrrolidone and imidazolimine methochloride; the terpolymer sold by Calgon in the USA under the trade name Merquat^{®} Plus 3300 made of dimethyldiallylammonium chloride, sodium acrylate, and acrylamide; the terpolymer sold by ISP in the USA under the trade name Gaffix^{®} VC 713, made of vinylpyrrolidone, dimethylaminoethyl methacrylate, and vinylcaprolactam; and the copolymer sold by Gaf under the trade name Gafquat^{®} HS 100 made of vinylpyrrolidone/methacrylamide propyltrimethylammonium chloride.

Suitable cationic polymers that are derived from natural polymers are the cationic derivatives of polysaccharides, for example, cationic derivatives of starch or guar. Furthermore, chitosan and chitosan derivatives are suitable.

Cationic polysaccharides have the general Formula

G-O-B-N⁺R^{a}R^{b}R^{c} X⁻ (V)

where G is an anhydroglucose residue, for example, starch or cellulose anhydroglucose; B is a divalent linking group, for example, alkyls, oxyalkyls, polyoxyalkyls, or hydroxyalkyls; R^{a}, R^{b}, and W are independently from one another alkyl, aryl, alkylaryl, arylalkyl, alkoxyalkyl, or alkoxyaryl each having 1 to 18 C atoms, wherein the entire number of C atoms in R^{a}, R^{b}, and R^{c} is preferably a maximum of 20; X ⁽⁻⁾ is a common anion, preferably chloride, bromide, or sulfate.

A suitable cationic cellulose of the Formula (V) is sold commercially under the name Polymer JR^{®} by the Amerchol Company and has the INCI designation POLYQUATERNIUM-10. An additional cationic cellulose bearing the INCI designation POLYQUATERNIUM-24 is sold commercially under the trade name Polymer LM^{®}-200 by Amerchol. A suitable cationic guar derivative is sold under the trade name Jaguar^{®} R and has the INCI designation GUAR HYDROXYPROPYL TRIMONIUM CHLORIDE.

Other suitable cationic polymers are chitosan, chitosan salts, and chitosan derivatives. The chitosans are completely or partially deacetylated chitins. To produce a chitosan, one preferably starts with the chitin contained in the shell residues of crustaceans, which is available in large quantities as a cheap and natural raw material. The molecular weight of chitosan can be distributed over a broad range, for example from 20,000 g/mol to about 5,000,000 g/mol (176,369.8 oz/mol). A low-molecular chitosan with a molecular weight of, for example, from 30,000 g/mol (1,058.2 oz/mol) to 70,000 g/mol (2,469.2 oz/mol) is suitable. However, the molecular weight preferably lies above 100,000 g/mol (3,527.39 oz/mol), and especially preferably from 200,000 g/mol (7,054.79 oz/mol) to 700,000 g/mol (24,691.8 oz/mol). The level of deacetylation is preferably from 10 to 99%, with 60 to 99% being especially preferred.

A suitable chitosan is sold, for example, by Kyowa Oil & Fat in Japan under the trade name Flonac^{®}. It has a molecular weight of from 300,000 g/mol (10,582.2 oz/mol) to 700,000 g/mol (24,691.8 oz/mol) and is 70 to 80% deacetylated. A preferred chitosan salt is chitosonium pyrrolidone carboxylate, which, for example, is sold under the name Kytamer PC by Amerchol Company in the USA. The chitosan contained has a molecular weight of from about 200,000 g/mol (7,054.79 oz/mol) to 300,000 g/mol (10,582.2 oz/mol) and is 70 to 85% deacetylated. Quaternized, alkylated, or hydroxyalkylated derivatives, for example, hydroxyethyl or hydroxybutyl chitosan can be considered chitosan derivatives.

The chitosans or chitosan derivatives are preferably present in their neutralized or partially neutralized form. The degree of neutralization for the chitosan or the chitosan derivative is preferably at least 50%, with 70 to 100% being especially preferred, based on the number of free base groups. All cosmetically compatible inorganic or organic acids can be used in principle for the neutralizing agent, such as for example formic acid, malic acid, succinic acid, tartaric acid, citric acid, malonic acid, oxalic acid and pyrrolidonecarboxylic acid, among which citric acid is preferred.

Other suitable cation-active, hair care compounds that can be contained in the shaping agent of the present invention are cationically-modified protein derivatives or cationically-modified protein hydrolysates, and they are known under the INCI designations Lauryldimonium Hydroxypropyl Hydrolyzed Wheat Protein, Lauryldimonium Hydroxypropyl Hydrolyzed Casein, Lauryldimonium Hydroxypropyl Hydrolyzed Collagen, Lauryldimonium Hydroxypropyl Hydrolyzed Keratin, Lauryldimonium Hydroxypropyl Hydrolyzed Silk, Lauryldimonium Hydroxypropyl Hydrolyzed Soy Protein, or Hydroxypropyltrimonium Hydrolyzed Wheat, Hydroxypropyltrimonium Hydrolyzed Casein, Hydroxypropyltrimonium Hydrolyzed Collagen, Hydroxypropyltrimonium Hydrolyzed Keratin, Hydroxypropyltrimonium Hydrolyzed Rice Bran Protein, Hydroxypropyltrimonium Hydrolyzed Silk, Hydroxypropyltrimonium Hydrolyzed Soy Protein, and Hydroxypropyltrimonium Hydrolyzed Vegetable Protein.

Suitable cationically-derived protein hydrolysates are mixtures of substances, which, for example, can be obtained from glycidyltrialkylammonium salts or 3-halo-2-hydroxypropyltrialkyl ammonium salts via the conversion of alkaline, acidic, or enzyme-hydrolyzed proteins. Proteins that are used as starting materials for the protein hydrolysates can be of plant or animal origin. Customary starting materials are, for example, keratin, collagen, elastin, soy protein, rice protein, milk protein, wheat protein, silk protein, or almond protein. Hydrolysis produces mixtures of materials with molecular masses in the range of from about 100 to about 50,000. Generally, the average molecular mass lies in the range of from about 500 to about 1,000. It is advantageous for the cationically-derivatized protein hydrolysates to contain one or two long C-8 to C-22 alkyl chains and correspondingly two or one short C-1 to C-4 alkyl chains. Compounds containing one long alkyl chain are preferred.

Suitable cationic polymers are also cation-active silicone compounds. These compounds are substituted with cationic groups or groups that can be made cationic. Suitable cation-active silicone compounds either have at least one amino group or at least one ammonium group. Suitable silicone polymers with amino groups are known under the INCI designation AMODIMETHICONE. These polymers are polydimethylsiloxanes with aminoalkyl groups. The polymer sold by the Dow Chemical Company under the trade name DC2-8556 (CTFA: AMODIMETHICONE) and the polymer sold by the General Electric Company subsidiary Osi under the trade name Silsoft A-858 (CTFA: DEA PG-PROPYL PEG/PPG-18/21 DIMETHICONE) are particularly suitable.

Especially preferred is when the permanent shaping agent of the present invention, in addition to the cationic cellulose derivative as recited in Claim 1, contains a synergistically-acting cationic polymer selected from among: diallyldimethylammonium chloride/hydroxyethylcellulose copolymers (CTFA: POLYQUATERNIUM-4), beta-methacryloxyethyltrimethylammonium methosulfate/acrylamide copolymers (CTFA: POLYQUATERNIUM-5), polydiallyldimethylammonium chloride (CTFA: POLYQUATERNIUM-6), for example as sold commercially by the Ondeo Nalco Company under the trade name Merquat^{®} 100, copolymers from acrylamide and dimethyldiallylammonium chloride (CTFA: POLYQUATERNIUM-7), quaternized vinylpyrrolidone/dimethylaminoethyl methacrylate copolymer (POLYQUATERNIUM-11), polydimethylaminoethyl methacrylate (75% quaternized with dimethylsulfate or 100% quaternized with methyl chloride or methyl bromide), beta-methacryloxyethyltrimethylammonium methosulfate homopolymer (CTFA: POLYQUATERNIUM-14), beta-methacrylyloxyethyltrimethylammonium chloride/methacrylamide copolymers (CTFA: POLYQUATERNIUM-15), methylvinylimidazolium chloride/vinylpyrrolidone copolymer (CTFA: POLYQUATERNIUM-16); N-vinylpyrrolidone/methacrylamide propyltrimethylammonium chloride copolymers, acrylic acid polymers such as for example the polymer sold commercially by the Röhm GmbH Company under the trade name PLEX 3074 L (CTFA: POLYQUATERNIUM-35), as well as cationic guar derivatives and cationic chitosan derivatives.

The shaping agent contains keratin-reducing active ingredients such as sulfites or mercapto compounds and especially thioglycolic acid, glycerin esters of thioglycolic acid, thioglycolic amides, thioglycerin, thiolactic acid, 3-mercaptopropionic acid, cysteine, cysteine derivatives such as for example cysteine-2-hydroxyethyl ester, cysteamine, homocysteine, alkyl or acyl cysteines, mercaptoacetamides, 2-mercaptopropionic acid amides or the salts or derivatives of these compounds, and especially ammonium thioglycolate. The keratin-reducing material is contained in the ready-to-use agent for permanent hair shaping in a quantity of from 1 to 25 percent by weight, with from 5 to 15 percent by weight being preferred.

It is advantageous if the permanent shaping agent also contains the disulfide of a hair keratin-reducing thiol, particularly dithioglycolate. The preferred usage quantity for the disulfide is from 1 to 20 percent by weight, but preferably from 2 to 10 percent by weight, wherein a ratio between the hair keratin-reducing compound and the disulfide of from 2 : 1 to 1 : 2, but particularly from 2 : 1 to 1 : 1, is preferred.

Obviously, the ready-to-use permanent shaping agent can contain all of the additives that are customary and known for such agents, for example thickeners such as for example fatty acids, hydroxyethylcelluloses, starches, vaseline, paraffin oils; wetting agents or emulsifiers from the classes of cationic, amphoteric or nonionic surface-active substances, such as quaternary ammonium salts, for example cetyltrimethylammonium chloride, N-alkylbetaines and N-alkylamidobetaines, N-alkylsulfobetaines, N-alkylaminopropionates, alkyldimethylcarboxymethylammonium salts with 12 to 18 carbon atoms as well as fatty acid alkylamidobetaines, for example fatty acid amidopropyldimethylaminoacetic acid betaines, oxyethylated alkyl phenols, fatty acid alkanolamides or oxyethylated fatty acid esters; moreover opacifiers, such as for example polyethylene glycol esters, alcohols, such as for example ethanol, propanol, isopropanol, polyols such as for example ethylene glycol, 1,2- or 1,3-propanediol, 1,2-, 1,3- or 1,4-butanediol, 1,2-, 1,3-, 1,4- or 1,5-pentanediol and glycerin; sugars such as for example D-glucose, solubilizing agents such as for example dipropylene glycol monomethyl ether, hydrogenated and oxyethylated caster oil, oxyethylated fatty alcohols, stabilizers, alkalizing agents, for example ammonia, mono- or diethanolamine, buffering agents, for example ammonium hydrogen carbonate, perfume oils, dyes as well as hair-conditioning and hair care components, such as for example lanolin derivatives, urea, cholesterol, pantothenic acid and betaine.

The components mentioned as being contained in this agent are used in usual amounts for such applications in a permanent shaping agent, for example the wetting agents and emulsifiers can be present in concentrations of from approximately 0.2 to 30 percent by weight, the alcohols can be present in an amount of from approximately 0.1 to 20 percent by weight, the opacifying agents, perfume oils and dyes can each be present in an amount of from 0.01 to 1 percent by weight, the buffering agents can be present in an amount of from approximately 0.1 to 10 percent by weight, sugars, solubilizing agents, stabilizers, as well as hair-conditioning and hair care component can each be present in an amount of from 0,1 to 5 percent by weight, while the thickeners and solubilizing agents can be present in an amount of from approximately 0.5 to 20 percent by weight.

Furthermore, this agent can contain so-called swelling and penetrating agents for the purpose of activity enhancement, such as for example dipropylene glycol monomethyl ether, 2-pyrrolidone or imidazolidin-2-one, in an amount of from 1 to 30 percent by weight.

The agent of the present invention effectively has a viscosity of from 1.5 to 5,000 mPa - s at 25 degrees Celsius (77 °F), wherein a viscosity of from 3 to 3,000 mPa · s at 25 degrees Celsius (77 °F) is preferred.

The pH value of the shaping agent is preferably from 7 to 10, wherein the pH is preferably adjusted using ammonia, monoethanolamine, ammonium carbonate, or ammonium hydrogen carbonate. If the shaping agent is adjusted to be acidic (for example to a pH = 6.5 to 6.9), esters of mercaptocarboxylic acids such as monothioglycolic acid glycol esters or -glycerin esters, but preferably mercaptoacetamides or 2-mercaptopropionic acid amides, are used in a concentration of from 2 to 14 percent by weight; or salts of sulfuric acid, for example, sodium, ammonium, or monoethanol ammonium sulfite are used in a concentration of from 3 to 8 percent by weight (calculated as SO₂).

Another object of the present invention is a method for the permanent shaping of hair in which the hair is treated with a hair keratin-reducing shaping agent before and/or after it is set into the desired shape; it is then rinsed with water, as necessary, after an action period that is sufficient for permanent shaping of the hair; the hair is then treated with an oxidative fixing agent, re-rinsed with water, styled, and then dried, wherein the aforementioned shaping agent of the present invention is used as the shaping agent.

In the process of the present invention, the hair is washed, towel-dried, separated into individual strands, and rolled onto curlers. The diameter of the curlers depends upon whether a permanent wave or uncrimping of the hair is desired, either 5 mm (0.2 in) to 13 mm (0.4 in) or about 15 mm (0.5 in) to 35 mm (1.2 in). A quantity of the shaping agent of the present invention that is sufficient for permanent hair shaping is applied to the hair before and/or after it is rolled onto curlers. The total quantity of the shaping agent required for the permanent hair shaping is generally from about 80 g (2.8 oz) to 120 g (4.23 oz).

After an action period that is sufficient for the permanent shaping of the hair, which can be from 5 to 30 minutes (10 to 30 minutes without the addition of heat or 5 to 20 minutes with heat) depending on hair quality, the shaping effectiveness of the shaping agent, as well as the application temperature, the hair is then rinsed with water and treated with an oxidative agent ("fixed"). Depending on the thickness of the hair, the fixing agent is used preferably in an amount of from 80 g (2.8 oz) to 100 g (3.53 oz).

Any fixing agent known to be suitable for this type of treatment can be used as the fixing agent for the hair, either rolled up in curlers or unrolled. Examples of oxidizing agents that can be used in such fixing agents include potassium and sodium bromate, sodium perborate, urea, and hydrogen peroxide. The concentration of the oxidizing agent can vary depending on the application time (normally 5 to 15 minutes) and the application temperature. The oxidizing agent is normally present in the ready-to-use aqueous fixing agent in a concentration of from 0.5 to 10 percent by weight. The fixing agent can obviously contain other materials, such as for example wetting agents, conditioning agents such as betaine, weak acids, buffering agents or peroxide stabilizers, and can be present in the form of an aqueous solution, an emulsion as well as in a thickened water-based form, especially as a creme, gel or paste. The usual additives can also be contained in the fixing agent in a quantity of from 0.1 to 10 percent by weight.

The curlers are then removed. If necessary, an oxidative post-treatment can be given to the unrolled hair. The hair is then rinsed with water, shaped into a water-wave as needed, and subsequently dried.

The hair treated in this manner has uniform and long-lasting shaping and is exceedingly well-conditioned.

The following examples are intended to provide additional details as to the object of the present invention.

### Examples

### Example 1 Run-resistant, mildly alkaline permanent wave agent for normal hair

| | |
|---|---|
| 18.20 g (0.642 oz) | ammonium thioglycolate (70% aqueous solution) |
| 2.00 g (0.07 oz) | ammonia (25% aqueous solution) |
| 4.00 g (0.14 oz) | ammonium hydrogen carbonate |
| 0.15 g (0.005 oz) | cellulose derivative of Formula (III) with a viscosity of 2,112 mPa · sec at 25 °C (77 °F) in a 1% aqueous solution (Amerchol Polymer SL®-60) |
| 0.20 g (0.007 oz) | polydimethyldiallylammonium chloride (CTFA: Polyquaternium-6) |
| 0.20 g (0.007 oz) | vinylpyrrolidone/dimethylaminoethyl methacrylate methosulfate copolymer (CTFA: Poiyquatemium-11) |
| 1.00 g (0.04 oz) | 1,3-butanediol |
| 1.50 g (0.05 oz) | 1,2-propylene glycol |
| 2.00 g (0.07 oz) | dipropylene glycol monoethyl ether |
| 3.50 g (0.12 oz) | urea |
| 1.00 g (0.04 oz) | hydrogenated castor triglyceride polyglycol ether (CTFA: PEG-40 Hydrogenated Castor Oil) |
| 1.00 g (0.04 oz) | coconut fatty alcohol, oxyethylated with 8 moles of ethylene oxide (CTFA: Coceth-8) |
| 0.50 g (0.02 oz) | perfume oil |
| balance to 100.00 g (3.53 oz) | water |

After the hair has been rolled up in permanent wave curlers, the aforementioned permanent wave agent is evenly applied to the curlers with the agent having a pH = 8.4 and a viscosity of 8.5 mPa · sec at 25 degrees Celsius (77 °F). There is then an action period of 10 minutes with the use of a hood type infrared radiator at a temperature of 40°C (104 °F). The hair is rinsed with water. Next, 80 g (2.8 oz) of one of the fixing agents mentioned below is applied to the curlers.

### Fixing agent

| | |
|---|---|
| 4.00 g (0.14 oz) | hydrogen peroxide, 50% |
| 0.10 g (0.004 oz) | salicylic acid |
| 0.20 g (0.007 oz) | disodium hydrogen phosphate |
| 0.15 g (0.005 oz) | o-phosphoric acid |
| 1.00 g (0.04 oz) | castor oil, oxyethylated with 35 moles of ethylene oxide |
| 0.10 g (0.004 oz) | vinylpyrrolidone/styrene copolymer |
| 0.10 g (0.004 oz) | perfume oil |
| balance to 100 g (3.53 oz) | water |

After an action period of 8 minutes, the fixing agent is rinsed out with water, and the hair is unrolled. The hair exhibits a conditioned appearance, a pleasant hold, and good combability when wet, as well as a uniform curl.

### Example 2 Run-resistant, mildly alkaline permanent wave agent for colored hair

| | |
|---|---|
| 12.50 g (0.44 oz) | ammonium thioglycolate (70% aqueous solution) |
| 2.00 g (0.07 oz) | diammonium dithioglycolate |
| 0.80 g (0.03 oz) | ammonia (25% aqueous solution) |
| 4.00 g (0.14 oz) | ammonium hydrogen carbonate |
| 0.15 g (0.005 oz) | cellulose derivative of Formula (III) with a viscosity of 2,112 mPa · sec at 25 °C (77 °F) in a 1% aqueous solution (Amerchol Polymer SL^{®}-60) |
| 0.80 g (0.03 oz) | polydimethyldiallylammonium chloride (CTFA: POLYQUATERNIUM-6) |
| 0.20 g (0.007 oz) | amino-functionalized silicone polymer (CTFA: METHOXY PEG/PPG-7/3 AMINOPROPYL DIMETHICONE) |
| 0.10 g (0.004 oz) | hydroxyethylcellulose |
| 0.30 g (0.01 oz) | styrene/vinylpyrrolidone copolymer (CTFA: STYRENE/VP COPOLYMER) |
| 1.50 g (0.05 oz) | 1,2-propylene glycol |
| 2.00 g (0.07 oz) | dipropylene glycol monoethyl ether |
| 2.00 g (0.07 oz) | urea |
| 1.00 g (0.04 oz) | hydrogenated castor triglyceride polyglycol ether (CTFA: PEG-40 Hydrogenated Castor Oil) |
| 1.00 g (0.04 oz) | coconut fatty alcohol, oxyethylated with 8 moles of ethylene oxide (CTFA: Coceth-8) |
| 0.50 g (0.02 oz) | perfume oil |
| balance to 100.00 g (3.53 oz) | water ( pH = 8.2) |

After the hair has been rolled up in permanent wave curlers, the aforementioned permanent wave agent is evenly applied to the curlers with the agent having a pH = 8.2 and a viscosity of 11 mPa · sec at 25 degrees Celsius (77 °F). There is then an action period of 20 minutes at room temperature. The hair is rinsed with water. Next, 80 g (2.8 oz) of the fixing agent from Example 1 is applied to the curlers. After an action period of 10 minutes, the fixing agent is rinsed out with water, and the hair is unrolled. The hair exhibits a conditioned appearance, a pleasant hold, and good combability when wet, as well as a uniform curl.

### Example 3 Run-resistant, mildly alkaline, permanent wave agent for curl-resistant hair

| | |
|---|---|
| 26.50 g (0.935 oz) | monoethanolamine thioglycolate (67% aqueous solution) |
| 3.80 g (0.13 oz) | monoethanolamine |
| 0.10 g (0.004 oz) | polymer of Formula (III) with a viscosity of 1,710 mPa · sec at 25 °C (77 °F) in a 1% aqueous solution (Amerchol Polymer SL^{®} 5) |
| 0.30 g (0.01 oz) | quaternized vinylpyrrolidone/dimethylaminoethyl methacrylate copolymer (POLYQUATERNIUM-11) |
| 0.02 g (0.0007 oz) | hydroxyethylcellulose |
| 1.00 g (0.04 oz) | 1,3-butanediol |
| 1.50 g (0.05 | oz) 1,2-propylene glycol |
| 2.00 g (0.07 oz) | dipropylene glycol monoethyl ether |
| 3.50 g (0.12 oz) | urea |
| 1.50 g (0.05 oz) | oleyl alcohol, oxyethylated with 10 moles of ethylene oxide (CTFA:OLETH-10) |
| 0.50 g (0.02 oz) | perfume oil |
| balance to 100.00 g (3.53 oz) | water (pH = 8.9) |

The permanent wave agent has a viscosity of 5.5 mPa · sec at 25 degrees Celsius (77 °F). The application is carried out according to Example 2. The hair exhibits a conditioned appearance, a pleasant hold, good combability, and a uniform curl.

### Example 4 Two-component, run-resistant permanent wave agent

### Component 1

| | |
|---|---|
| 1.50 g (0.05 oz) | ammonium hydrogen carbonate |
| 2.20 g (0.08 oz) | ammonia (25% aqueous solution) |
| 0.10 g (0.004 oz) | polymer of Formula (III) with a viscosity of 1,895 mPa · sec at 25 °C (77 °F) in a 1% aqueous solution (Amerchol Polymer SL^{®}-30) |
| 0.10 g (0.004 oz) | polymer of Formula (III) with a viscosity of 2,205 mPa · sec at 25 °C (77 °F) in a 1% aqueous solution (Amerchol Polymer SL^{®}-100) |
| 0.40 g (0.01 oz) | methylvinylimidazolium chloride/vinylpyrrolidone copolymer (CTFA: POLYQUATERNIUM-16) |
| 0.30 g (0.01 oz) | cetyltrimethylammonium chloride |
| 1.50 g (0.05 oz) | 1,2-propylene glycol |
| 3.00 g (0.11 oz) | urea |
| 1.80 g (0.06 oz) | oleyl alcohol, ethoxylated with 20 moles of ethylene oxide (CTFA: OLETH-20) |
| 0.40 g (0.01 oz) | perfume oil |
| balance to 100.00 g (3.53 oz) | water |
| *Component 2* | |
| 80.00 g (2.82 oz) | ammonium thioglycolate (70% aqueous solution) |
| 20.00 g (0.71 oz) | ammonium thiolactate (70% aqueous solution) |

Prior to use, 60 g (2.1 oz) of Component 1 was mixed with 15 g (0.5 oz) Component 2. The pH value of the ready-to-use hair shaping agent obtained was 7.3. Next, 75 g (2.6 oz) of the ready-to-use agent was applied to the hair that had previously been rolled around permanent wave curlers with a diameter of 8 mm (0.3 in), and this was allowed to act on the hair for 20 minutes at room temperature. The hair is then rinsed with water. Next, 80 g (2.8 oz) of the fixing agent from Example 1 was applied to the curlers. After an action period of 10 minutes, the fixing agent was rinsed out with water, and the hair was unrolled. The hair exhibited a conditioned appearance, a pleasant hold, and good combability when wet, as well as a uniform curl.

### Example 5 Gel-type permanent wave agent for root perms

| | |
|---|---|
| 18.20 g (0.642 oz) | ammonium thioglycolate (70% aqueous solution) |
| 2.20 g (0.09 oz) | ammonia (25% aqueous solution) |
| 3.50 g (0.12 oz) | ammonium hydrogen carbonate |
| 0.30 g (0.01 oz) | polymer of Formula (III) with a viscosity of 2,112 mPa · sec at 25 °C (77 °F) in a 1% aqueous solution (Amerchol Polymer SL^{®}-60) |
| 0.20 g (0.007 oz) | polydimethyldiallylammonium chloride (CTFA: POLYQUATERNIUM-6) |
| 0.80 g (0.03 oz) | hydroxyethylcellulose |
| 0.50 g (0.02 oz) | 1,3-butanediol |
| 2.00 g (0.07 oz) | 1,2-propylene glycol |
| 2.00 g (0.07 oz) | dipropylene glycol monoethyl ether |
| 3.50 g (0.12 oz) | urea |
| 1.50 g (0.05 oz) | polyethylene glycol castor oil (35EO) (CTFA: PEG-35 CASTOR OIL) |
| 1.00 g (0.04 oz) | hydrogenated castor triglyceride polyglycol ether (40EO) (CTFA: PEG-40 HYDROGENATED CASTOR OIL TRIISOSTEARATE) |
| 0.50 g (0.02 oz) | perfume oil |
| balance to 100.00 g (3.53 oz) | water (pH = 8.6) |

After the hair has been rolled up in permanent wave curlers, the aforementioned permanent wave agent is evenly applied to the hair roots with a brush with the agent having a pH = 8.6 and a viscosity of 2,700 mPa · sec at 25 degrees Celsius (77 °F). There is then an action period of 10 minutes at room temperature. The hair is rinsed with water. Next, 60 g (2.1 oz) of the fixing agent from Example 1 was applied to the curlers. After an action period of 10 minutes, the fixing agent is rinsed out with water, and the hair is unrolled. The hair exhibits good root volume, an excellent hold, and good combability.

### Example 6 Two-phase permanent wave agent to even out large structural differences of hair

### Phase 1

| | |
|---|---|
| 12.50 g (0.441 oz) | ammonium thioglycolate (70% aqueous solution) |
| 0.80 g (0.03 oz) | ammonia (25% aqueous solution) |
| 4.00 g (0.14 oz) | ammonium hydrogen carbonate |
| 0.10 g (0.004 oz) | Polymer of Formula (III) with a viscosity of 2.112 mPa - sec at 25 °C (77 °F) in a 1% aqueous solution (Amerchol Polymer SL^{®}-60) |
| 0.10 g (0.004 oz) | Polymer of Formula (III) with a viscosity of 2205 mPa · sec at 25 °C (77 °F) in a 1% aqueous solution (Amerchol Polymer SL^{®}-100) |
| 0.80 g (0.03 oz) | aminosilicone DC2-8566 from Dow Coming (CTFA: AMODIMETHICONE) |
| 0.30 g (0.01 oz) | styrene/vinylpyrrolidone copolymer (CTFA: STYRENE/VP COPOLYMER) |
| 1.50 g (0.05 oz) | 1,2-propylene glycol |
| 2.00 g (0.07 oz) | urea |
| 1.00 g (0.04 oz) | coconut fatty alcohol, ethoxylated with 8 moles of ethylene oxide (CTFA: COCETH-8) |
| 1.00 g (0.04 oz) | hydrogenated castor triglyceride polyglycol ether (40EO) (CTFA: PEG-40 HYDROGENATED CASTOR OIL TRIISOSTEARATE) |
| 0.50 g (0.02 oz) | perfume oil |
| balance to 100.00 g (3.53 oz) | water (pH = 8.2) |

### Phase 2

| | |
|---|---|
| 18.20 g (0.642 oz) | ammonium thioglycolate (70% aqueous solution) |
| 2.20 g (0.08 oz) | ammonia (25% aqueous solution) |
| 3.50 g (0.12 oz) | ammonium hydrogen carbonate |
| 0.40 g (0.01 oz) | polymer of Formula (III) with a viscosity of 2,112 mPa · sec at 25 °C (77 °F) in a 1% aqueous solution (Amerchol Polymer SL^{®}-60) |
| 0.20 g (0.007 oz) | polydimethyldiallylammonium chloride (CTFA: POLYQUATERNIUM-6) |
| 0.80 g (0.03 oz) | hydroxyethylcellulose |
| 0.50 g (0.02 oz) | 1,3-butanediol |
| 2.00 g (0.07 oz) | 1,2-propylene glycol |
| 2.00 g (0.07 oz) | dipropylene glycol.monoethyl ether |
| 3.50 g (0.12 oz) | urea |
| 1.50 g (0.05 oz) | polyethylene glycol castor oil (35EO) (CTFA: PEG-35 CASTOR OIL) |
| 1.00 g (0.04 oz) | hydrogenated castor triglyceride polyglycol ether (40EO) (CTFA: PEG-40 HYDROGENATED CASTOR OIL TRIISOSTEARATE) |
| 0.50 g (0.02 oz) | perfume oil |
| balance to 100.00 g (3.53 oz) | water (pH = 8.6) |

After the hair has been rolled up in permanent wave curlers, the liquid permanent wave agent from Phase 1 is then applied to the curlers with the agent having a pH = 8.2 and a viscosity of 7 mPa · sec at 25 degrees Celsius (77 °F). Immediately afterwards, the above mentioned gel-type permanent wave agent (Phase 2) is applied to the hair roots with a brush, where said agent has a pH = 8.6 and a viscosity of 2,900 mPa · sec at 25 degrees Celsius (77 °F). There is then an action period of 10 minutes at room temperature. The hair is then rinsed with water. Next, 60 g (2.1 oz) of the fixing agent from Example 1 was applied to the curlers. After an action period of 10 minutes, the fixing agent is rinsed out with water, and the hair is unrolled. The hair exhibits a uniform wave from the roots to the tips, excellent hold, and very good combability.

### Example 7 Run-resistant, mildly alkaline permanent wave agent for normal hair

| | |
|---|---|
| 18.20 g | ammonium thioglycolate (70% aqueous solution) |
| (0.64 oz) | |
| 2.00 g | ammonia (25% aqueous solution) |
| (0.07 oz) | |
| 4.00 g | ammonium hydrogen carbonate |
| (0.14 oz) | |
| 0.15 g | polymer of the Formula (IV) with a viscosity of |
| (0.0053 oz) | 2,112 mPa · sec at 25 °C (77 °F) in a 1% aqueous solution |
| 0.20 g | polydimethyl diallyl ammonium chloride |
| (0.0071 oz) | (CTFA: POLYQUATERNIUM-6) |
| 0.20 g | vinyl pyrrolidone/dimethylaminoethyl methacrylate methosulfate |
| (0.0071 oz) | copolymer(CTFA: POLYQUATERNIUM- 11) |
| 1.00 g | 1,3-butanediol |
| (0.035 oz) | |
| 1.50 g | 1,2-propyleneglycol |
| (0.053 oz) | |
| 2.00 g | dipropyleneglycol monoethyl ether |
| (0.071 oz) | |
| 3.50 g | urea |
| (0.12 oz) | |
| 1.00 g | hydrogenated ricinus triglyceride polyglycol ether |
| (0.035 oz) | (CTFA: PEG-40 HYDROGENATED CASTOR OIL) |
| 1.00 g | coconut fatty alcohol, oxyethylated with 8 mole ethylene oxide |
| (0.035 oz) | (CTFA: COCETH-8) |
| 0.50 g | perfume oil |
| (0.0 18 oz) | |
| balance to 100.00 g | water |
| (3.53 oz) | |

After the hair has been rolled up in permanent wave curlers, the aforementioned permanent wave agent is evenly applied to the curlers with the agent having a pH = 8.4 and a viscosity of 8.5 mPa · sec at 25 degrees Celsius (77 °F). There is then an action period of 10 minutes with the use of a hood type infrared radiator at a temperature of 40 °C (104 °F). The hair is rinsed with water. 80 g (2.82 oz) of the fixing agent of Example 1 is then applied to the curlers.

After an action period of 8 minutes, the fixing agent is rinsed out with water, and the hair is unrolled. The hair exhibits a conditioned appearance, a pleasant hold, and good combability when wet, as well as a uniform curl.

### Example 8 Run-resistant, mildly alkaline permanent wave agent for colored hair

| | |
|---|---|
| 12.50 g | ammonium thioglycolate (70% aqueous solution) |
| (0.44 oz) | |
| 2.00 g | diammonium dithioglycolate |
| (0.071 oz) | |
| 0.80 g | ammonia (25% aqueous solution) |
| (0.028 oz) | |
| 4.00 g | ammonium hydrogen carbonate |
| (0. 14 oz) | |
| 0.15 g | polymer of the Formula (IV) with a viscosity of |
| (0.0053 oz) | 2,112 mPa · sec at 25 °C (77 °F) in a 1% aqueous solution |
| 0.80 g | polydimethyl diallyl ammonium chloride |
| (0.028 oz) | (CTFA: POLYQUATERNIUM-6) |
| 0.20 g | amino-functional silicon polymer (CTFA: METHOXY PEG/PPG-7/3 |
| (0.0071 oz) | AMINOPROPYL DIMETHICONE) |
| 0.10 g | hydroxyethyl cellulose |
| (0.0035 oz) | |
| 0.30 g | styrene/vinyl pyrrolidone copolymer |
| (0.012 oz) | (CTFA: STYRENE/VP COPOLYMER) |
| 1.50 g | 1,2-propyleneglycol |
| (0.053 oz) | |
| 2.00 g | dipropyleneglycol monoethyl ether |
| (0.071 oz) | |
| 2.00 g | urea |
| (0.071 oz) | |
| 1.00 g | hydrogenated ricinus triglyceride polyglycol ether |
| (0.035 oz) | (CTFA: PEG-40 HYDROGENATED CASTOR OIL) |
| 1.00 g | coconut fatty alcohol, oxyethylated with 8 mole ethylene oxide |
| (0.035 oz) | (CTFA: COCETH-8) |
| 0.50 g | perfume oil |
| (0.018 oz) | |
| balance to 100.00 g | water (pH = 8.2) |
| (3.53 oz) | |

After the hair has been rolled up in permanent wave curlers, the aforementioned permanent wave agent is evenly applied to the curlers with the agent having a pH = 8.2 and a viscosity of 11 mPa · sec at 25 degrees Celsius (77 °F). There is then an action period of 20 minutes at room temperature. The hair is rinsed with water. 80 g (2.82 oz) of the fixing agent from Example 1 is then applied to the curlers. After an action period of 10 minutes, the fixing agent is rinsed out with water, and the hair is unrolled. The hair exhibits a conditioned appearance, a pleasant hold, and good combability when wet, as well as a uniform curl.

### Example 9 Gel-type permanent wave agent for root perms

| | |
|---|---|
| 18.20 g | ammonium thioglycolate (70% aqueous solution) |
| (0.64 oz) | |
| 2.20 g | ammonia (25% aqueous solution) |
| (0.078 oz) | |
| 3.50 g | ammonium hydrogen carbonate |
| (0.12 oz) | |
| 0.30 g | polymer of the Formula (IV) with a viscosity of |
| (0.011 oz)) | 2,112 mPa · sec at 25 °C (77 °F) in a 1% aqueous solution |
| 0.20 g | polydimethyl diallyl ammonium chloride |
| (0.0071 oz) | (CTFA: POLYQUATERNIUM-6) |
| 0.80 g | hydroxyethyl cellulose |
| (0.028 oz) | |
| 0.50 g | 1,3-butanediol |
| (0.018 oz) | |
| 2.00 g | 1,2-propyleneglycol |
| (0.071 oz) | |
| 2.00 g | dipropyleneglycol monoethyl ether |
| (0.071 oz) | |
| 3.50 g | urea |
| (0. 12 oz) | |
| 1.50 g | polyethylene glycol castor oil(35EO) |
| (0.053 oz) | (CTFA: PEG-35 CASTOR OIL) |
| 1.00 g | hydrogenated ricinus triglyceride polyglycol ether(40EO) |
| (0.035 oz) | (CTFA: PEG-40 HYDROGENATED CASTOR OIL TRIISOSTEARATE) |
| 0.50 g | perfume oil |
| (0.018 oz) | |
| balance to 100.00 g | water (pH = 8.6) |
| (3.53 oz) | |

After the hair has been rolled up in permanent wave curlers, the aforementioned permanent wave agent is evenly applied to the hair roots with a brush with the agent having a pH = 8.6 and a viscosity of 2,700 mPa · sec at 25 degrees Celsius (77 °F). There is then an action period of 10 minutes at room temperature. The hair is rinsed with water. 60 g (2.12 oz) of the fixing agent from Example 1 is then applied to the curlers. After an action period of 10 minutes, the fixing agent is rinsed out with water, and the hair is unrolled. The hair exhibits good root volume, an excellent hold, and good combability.

### Example 10 2-Phase permanent wave agent to even out large structural differences of hair

### Phase 1

| | |
|---|---|
| 12.50 g | ammonium thioglycolate (70% aqueous solution) |
| (0.44 oz) | |
| 0.80 g | ammonia (25% aqueous solution) |
| (0.028 oz) | |
| 4.00 g | ammonium hydrogen carbonate |
| (0.14 oz) | |
| 0.10 g | polymer of the Formula (IV) with a viscosity of |
| (0.0035 oz) | 2,112 mPa · sec at 25 °C (77 °F) in a 1% aqueous solution |
| 0.10 g | polymer of the Formula (IV) with a viscosity of |
| (0.0035 oz) | 2,205 mPa · sec at 25 °C (77 °F) in a 1% aqueous solution |
| 0.80 g | aminosilicon DC2-8566 from Dow Coming |
| (0.028 oz) | (CTFA: AMODIMETHICONE) |
| 0.30 g | styrene/vinyl pyrrolidone copolymer |
| (0.011 oz) | (CTFA: STYRENE/VP COPOLYMER) |
| 1.50g | 1,2-propyleneglycol |
| (0.053 oz) | |
| 2.00 g | urea |
| (0.071 oz) | |
| 1.00 g | coconut fatty alcohol, ethoxylated with 8 mole ethylene oxide |
| (0.0350z) | (CTFA: COCETH-8) |
| 1.00 g | hydrogenated ricinus triglyceride polyglycol ether(40EO) |
| (0.035 oz) | (CTFA: PEG-40 HYDROGENATED CASTOR OIL TRIISOSTEARATE) |
| 0.50 g | perfume oil |
| (0.018 oz) | |
| balance to 100.00 g | water (pH = 8.2) |
| (3.53 oz) | |

### Phase 2

| | |
|---|---|
| 18.20 g | ammonium thioglycolate (70% aqueous solution) |
| (0.64 oz) | |
| 2.20 g | ammonia (25% aqueous solution) |
| (0.078 oz) | |
| 3.50 g | ammonium hydrogen carbonate |
| (0.12 oz) | |
| 0.40 g | polymer of the Formula (IV) with a viscosity of |
| (0.014 oz) | 2,112 mPa · sec at 25 °C (77 °F) in a 1% aqueous solution |
| 0.20 g | polydimethyl diallyl ammonium chloride |
| (0.0071 oz) | (CTFA: POLYQUATERNIUM-6) |
| 0.80 g | hydroxyethyl cellulose |
| (0.028 oz) | |
| 0.50 g | 1,3-btanediol |
| (0.018 oz) | |
| 2.00 g | 1,2-propyleneglycol |
| (0.071 oz) | |
| 2.00 g | dipropyleneglycol monoethyl ether |
| (0.071 oz) | |
| 3.50 g | urea |
| (0.12 oz) | |
| 1.50 g | polyethylene glycol castor oil (35EO) |
| (0.053 oz) | (CTFA: PEG-35 CASTOR OIL) |
| 1.00 g | hydrogenated ricinus triglyceride polyglycol ether (40EO) |
| (0.035 oz) | (CTFA: PEG-40 HYDROGENATED CASTOR OIL TRIISOSTEARATE) |
| 0.50 g | perfume oil |
| (0.0 18 oz) | |
| balance to 100.00 | g water (pH = 8.6) |
| (3.53 oz) | |

After the hair has been rolled up in permanent wave curlers, the liquid permanent wave agent from Phase 1 is then applied to the curlers with the agent having a pH = 8.2 and a viscosity of 7mPa · sec at 25 degrees Celsius (77 °F). Immediately afterwards, the aforementioned gel-type permanent wave agent is applied to the hair roots with a brush with the agent having a pH = 8.6 and a viscosity of 2,900 mPa · sec at 25 degrees Celsius (77 °F). There is then an action period of 10 minutes at room temperature. The hair is then rinsed with water. 60 g (2.12 oz) of the fixing agent from Example 1 is then applied to the curlers. After an action period of 10 minutes, the fixing agent is rinsed out with water, and the hair is unrolled. The hair exhibits a uniform wave from the hairline to the hair tips, an excellent hold, and very good combability.

Unless indicated otherwise, all percentages given in the present application represent weight percents.

All viscosity values are based on the measurement with a Haake model VT 550 rotational viscometer, with a shear rate of 10.0 per second. A model NV or MV double-gap cylinder measuring system was used.

## Claims

1. A hair keratin-reducing shaping agent for carrying out the reductive step in a permanent hair shaping, comprising at least one hair keratin-reducing compound and a cationic cellulose derivative that is formed from a cellulose ether with from 1,000 to 10,000 repeating anhydroglucose units, which (a) is substituted with 0.0001 to 0.1 moles per anhydroglucose unit of a substituent of Formula
**(I)** **R¹R²R³R⁴N⁺ (A^{z-}) _{1/z}**
where,
R¹ and R² independently from one another stand for -CH₃ or -C₂H₅,
R³ stands for -CH₂-CH(OH)-CH₂- or -CH₂CH₂-,
R⁴ represents a straight-chain or branched alkyl residue with 6 to 24 carbon atoms, A^{z-} is an anion, selected from among chloride, sulfate, nitrate or phosphate, and
z signifies 1, 2 or 3, and
(b) is substituted with 0.01 to 0.9 moles per anhydroglucose unit of a substituent of Formula
**(II**) **R⁵R⁶R⁷R⁸N⁺ (A^{z-}) _{1/z}**
where,
R⁵, R⁶ and R⁷ independently from one another stand for -CH₃ or -C₂H₅,
R⁸ stands for -CH₂-CH(OH)-CH₂- or -CH₂CH₂-,
A^{z-} is an anion, selected from among chloride, sulfate, nitrate or phosphate, and
z signifies 1, 2 or 3.

2. The agent as recited in Claim 1, wherein in Formula (I), R¹ and R² stand for -CH₃ and R³ stands for -CH₂-CH(OH)CH₂-.

3. The agent as recited in either of Claims 1 or 2, wherein in Formula (I), R⁴ stands for a straight-chain or branched alkyl residue with 10 to 16 carbon atoms.

4. The agent as recited in any of Claims 1 through 3, wherein in Formula (II), R⁵, R⁶ and R⁷ stand for -CH₃.

5. The agent as recited in any of Claims 1 through 4, wherein in Formula (II), R⁸ stands for -CH₂-CH(OH)-CH₂-.

6. The agent as recited in any of Claims 1 through 5, wherein said agent contains a cationic cellulose derivative of the general formula (III), 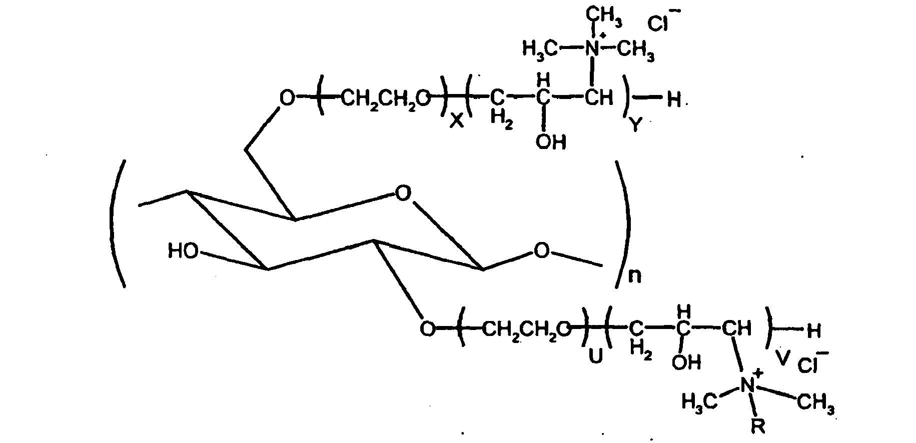 where,
X stands for a number from 0.05 to 0.8,
Y is a number from 0.05 to 0.8,
U stands for a number from 0.0005 to 0.05,
V stands for a number from 0.0005 to 0.05,
R is a straight-chain or branched alkyl residue with 6 to 18 carbon atoms and n stands for an integer from 1,000 to 8,000.

7. The agent as recited in Claim 6, wherein in Formula (III) R = C₁₂ H₂₅.

8. The agent as recited in Claims 1, wherein said agent contains a cationic cellulose derivative of the general formula (IV), 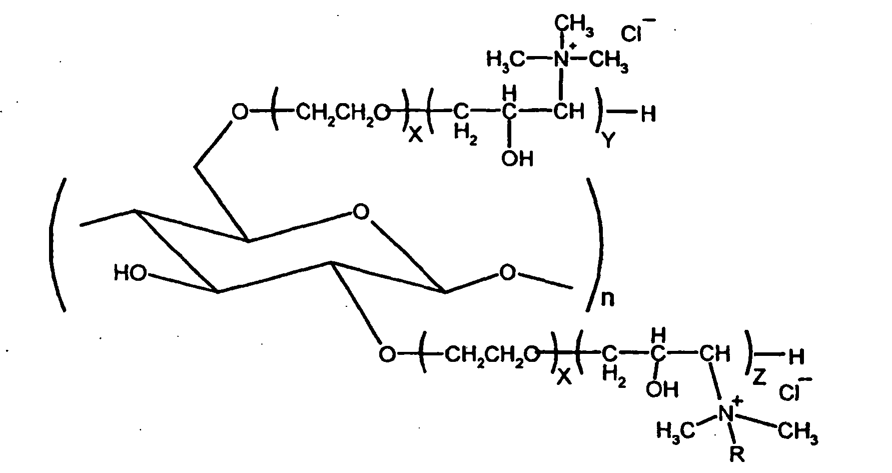 wherein,
X means a whole number of from 1 to 10,
Y is a whole number of from 0 to 10,
Z means a whole number of from 1 to 10,
R is a straight-chain or branched alkyl group with 6 to 18 carbon atoms, and
n means a whole number of from 10 to 5,000.

9. Agent as recited in Claim 8, **characterized in that**, in the Formula (IV),
X means a whole number of from 1 to 4,
Y means a whole number of from 1 to 4,
Z means a whole number of from 1 to 4,
R is a straight-chain alkyl group with 10 to 16 carbon atoms, and
n means a whole number of from 50 to 1,000.

10. The agent as recited in Claim 8 or 9, wherein in Formula (IV) R = C₁₂ H₂₅.

11. The agent as recited in any of Claims 1 through 10, wherein a 1% aqueous solution of the cationic cellulose derivative exhibits a viscosity of 1,500 to 3,000 mPa · sec at 25 Grad Celsius (77 °F), measured with a Haake Model VT 550 rotational viscometer at a shear rate of 10.0 per second.

12. The agent as recited in any of Claims 1 through 11, wherein the cationic cellulose derivative is contained in an amount of from 0.1 to 5 percent by weight.

13. The agent as recited in any of Claims 1 through 12, wherein said agent additionally contains at least one cationic polymer that is selected from among poly(dimethylaminoethyl methacrylate) (up to 75% quaternized with dimethyl sulfate or to 100% with methyl chloride or methyl bromide), poly(diallyldimethylammonium chloride), diallyldimethylammonium chloride/hydroxyethylcellulose copolymers, beta-methacryloxyethyltrimethylammonium methosulfate homopolymer, beta-methacryloxyethyltrimethylammonium methosulfate/acrylamide copolymers, beta methacrylyloxyethyltrimethylammonium chloride/methacrylamide copolymers, copolymers of acrylamide and dimethyldiallylammonium chloride, vinylpyrrolidone/dimethylaminoethyl methacrylate copolymer, beta-methacryloxyethyltrimethylammonium methosulfate/vinylpyrrolidone copolymers, methylvinylimidazolium chloride/vinylpyrrolidone copolymer, diallyldimethylammonium chloride/acrylamide copolymers, N-vinylpyrrolidone/methacrylamidopropyldimethylammonium chloride copolymers, cationic chitosan derivatives, cationic cellulose derivatives different from the cationic cellulose derivatives of Claim 1 and cationic guar derivatives.

14. The agent as recited in any of Claims 1 through 13, wherein the total quantity of cationic polymer is 0.2 to 5 percent by weight.

15. The agent as recited in any of Claims 1 through 14, wherein the hair keratin-reducing compound is selected from among mercaptoacetic acid, cysteine, or thiolactic acid, or their salts.

16. The agent as recited in any of Claims 1 through 15, wherein the hair keratin-reducing compound in the wave shaping agent is contained in a quantity of from 2 to 15 percent by weight.

17. The agent as recited in any of Claims 1 through 16, wherein the wave shaping agent contains the disulfide of a hair keratin-reducing compound.

18. The agent as recited in Claim 17, wherein the disulfide of the hair keratin-reducing compound is dithioglycolate.

19. The agent as recited in either of Claims 17 or 18, wherein the disulfide of the hair keratin-reducing compound is contained in a quantity of from 2 to 20 percent by weight.

20. The agent as recited in any of Claims 17 through 19, wherein the ratio of the hair keratin-reducing compound to the disulfide of the hair keratin-reducing compound is from 2 : 1 to 1: 1.

21. A method for permanent hair shaping in which the hair is treated with a hair keratin-reducing shaping agent before and/or after the hair has been set in a desired form, and after an action period sufficient for the permanent shaping, the hair is rinsed with water if necessary, then the hair is given an oxidative post-treatment, and rinsed again with water, set in a hairstyle and finally dried, wherein an agent as recited in any of Claims 1 through 20 is used as the hair keratin-reducing shaping agent.

## Patentansprüche

1. Haarkeratin reduzierendes Formungsmittel zum Ausführen des reduktiven Schrittes bei einer permanenten Haarformung, umfassend mindestens eine Haarkeratin reduzierende Verbindung und ein kationisches Cellulosederivat, das aus einem Celluloseether mit 1.000 bis 10.000 Anhydroglucose-Grundeinheiten gebildet ist, der (a) mit 0,0001 bis 0,1 Mol pro Anhydroglucose-Einheit mit einem Substituenten der folgenden Formel substituiert ist
(I) R¹R²R³R⁴N⁺ (A^{z-}) _{1/z}
worin,
R¹ und R² unabhängig voneinander für -CH₃ oder -C₂H₅ stehen,
R³ für -CH₂-CH(OH)-CH₂- oder - CH₂CH₂- steht,
R⁴ für einen geradkettigen oder verzweigten Alkylrest mit 6 bis 24 Kohlenstoffatomen steht,
A^{z-} ein Anion ist, das aus Chlorid, Sulfat, Nitrat oder Phosphat ausgewählt ist, und
z für 1, 2 oder 3 steht, und
(b) mit 0,01 bis 0,9 Mol pro Anhydroglucose-Einheit mit einem Substituenten der folgenden Formel substituiert ist
(I-I) R⁵R⁶R⁷R⁸N⁺ (A^{z-}) _{1/z}
worin,
R⁵, R⁶ und R⁷ unabhängig voneinander für -CH₃ oder -C₂H₅ stehen,
R⁸ für -CH₂-CH(OH)-CH₂- oder - CH₂CH₂- steht,
A^{z-} ein Anion ist, das aus Chlorid, Sulfat, Nitrat oder Phosphat ausgewählt ist, und
z für 1, 2 oder 3 steht.

2. Mittel nach Anspruch 1, wobei in Formel (I) R¹ und R² für -CH₃ stehen und R³ für -CH₂-CH(OH)-CH₂- steht.

3. Mittel nach einem der Ansprüche 1 oder 2, wobei in Formel (I) R⁴ für einen geradkettigen oder verzweigten Alkylrest mit 10 bis 16 Kohlenstoffatomen steht.

4. Mittel nach einem der Ansprüche 1 bis 3, wobei in Formel (II) R⁵, R⁶ und R⁷ für -CH₃ stehen.

5. Mittel nach einem der Ansprüche 1 bis 4, wobei in Formel (II) R⁸ für -CH₂-CH(OH)-CH₂- steht.

6. Mittel nach einem der Ansprüche 1 bis 5, wobei das Mittel ein kationisches Cellulosederivat der folgenden allgemeinen Formel (III) enthält, 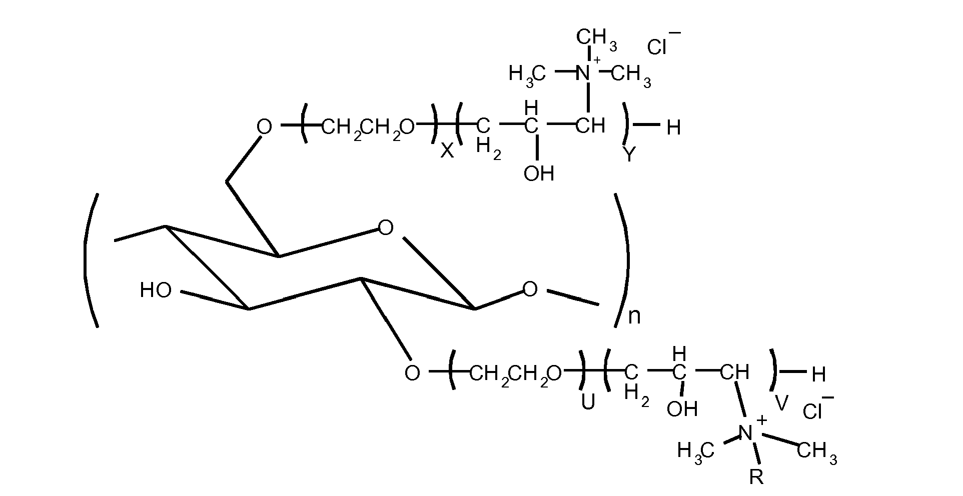 worin
X für eine Zahl von 0,05 bis 0,8 steht,
Y eine Zahl von 0,05 bis 0,8 ist,
U für eine Zahl von 0,0005 bis 0,05 steht,
V für eine Zahl von 0,0005 bis 0,05 steht,
R ein geradkettiger oder verzweigter Alkylrest mit 6 bis 18 Kohlenstoffatomen ist und
n für eine ganze Zahl von 1.000 bis 8.000 steht.

7. Mittel nach Anspruch 6, wobei in Formel (III) R = C₁₂ H₂₅.

8. Mittel nach Anspruch 1, wobei das Mittel ein kationisches Cellulosederivat der allgemeinen Formel (IV) enthält, 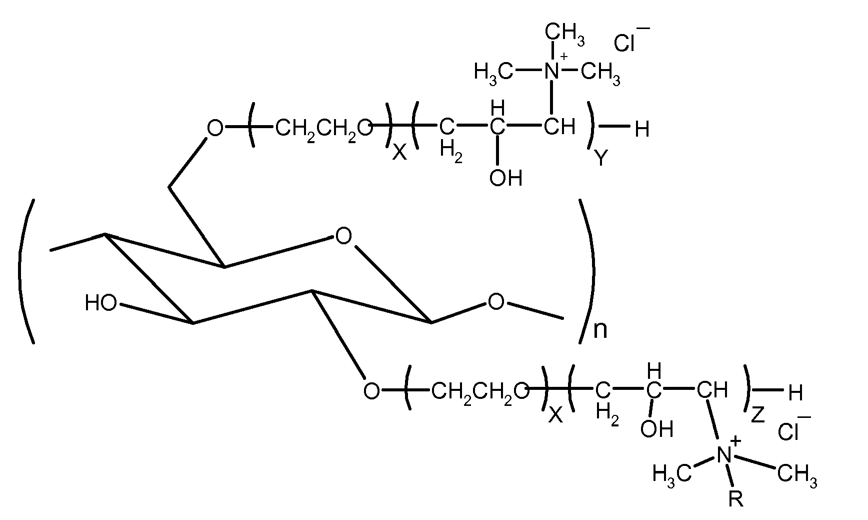 worin
X für eine ganze Zahl von 1 bis 10 steht,
Y eine ganze Zahl von 0 bis 10 ist,
Z für eine ganze Zahl von 1 bis 10 steht,
R eine geradkettige oder verzweigte Alkylgruppe mit 6 bis 18 Kohlenstoffatomen ist und
n für eine ganze Zahl von 10 bis 5.000 steht.

9. Mittel nach Anspruch 8, **dadurch gekennzeichnet, dass** in der Formel (IV)
X für eine ganze Zahl von 1 bis 4 steht,
Y für eine ganze Zahl von 1 bis 4 steht,
Z für eine ganze Zahl von 1 bis 4 steht,
R eine geradkettige Alkylgruppe mit 10 bis 16 Kohlenstoffatome ist und
n für eine ganze Zahl von 50 bis 1.000 steht.

10. Mittel nach Anspruch 8 oder 9, wobei in Formel (IV) R = C₁₂ H₂₅.

11. Mittel nach einem der Ansprüche 1 bis 10, wobei eine 1 %-ige wässrige Lösung des kationischen Cellulosederivats eine Viskosität von 1.500 bis 3.000 mPa·s bei 25 Grad Celsius (77 °F), gemessen mit einem Rotationsviskosimeter Haake Modell VT 550 bei einer Schergeschwindigkeit von 10,0 pro Sekunde, aufweist.

12. Mittel nach einem der Ansprüche 1 bis 11, wobei das kationische Cellulosederivat in einer Menge von 0,1 bis 5 Gewichtsprozent enthalten ist.

13. Mittel nach einem der Ansprüche 1 bis 12, wobei das Mittel zusätzlich mindestens ein kationisches Polymer enthält, das aus Poly(dimethylaminoethylmethacrylat) (bis zu 75 % mit Dimethylsulfat oder bis 100 % mit Methylchlorid oder Methylbromid quaternisiert), Poly(diallyldimethylammoniumchlorid), Diallyldimethylammoniumchlorid/Hydroxyethylcellulose-Copolymeren, beta-Methacryloxyethyltrimethylammoniummethosulfat-Homopolymer, beta-Methacryloxyethyltrimethylammoniummethosulfat/Acrylamid-Copolymeren, beta-Methacrylyloxyethyltrimethylammoniumchlorid/Methacrylamid-Copolymeren, Copolymeren von Acrylamid und Dimethyldiallylammoniumchlorid, Vinylpyrrolidon/Dimethylaminoethylmethacrylat-Copolymer, beta-Methacryloxyethyltrimethylammoniummethosulfat/Vinylpyrrolidon-Copolymeren, Methylvinylimidazoliumchlorid/Vinylpyrrolidon-Copolymer, Diallyldimethylammoniumchlorid/Acrylamid-Copolymeren, N-Vinylpyrrolidon/Methacrylamidopropyldimethylammoniumchlorid-Copolymeren, kationischen Chitosanderivaten, kationischen Cellulosederivaten, die sich von den kationischen Cellulosederivaten nach Anspruch 1 unterscheiden, und kationischen Guargummiderivaten ausgewählt ist.

14. Mittel nach einem der Ansprüche 1 bis 13, wobei die Gesamtmenge an kationischem Polymer 0,2 bis 5 Gewichtsprozent beträgt.

15. Mittel nach einem der Ansprüche 1 bis 14, wobei die Haarkeratin reduzierende Verbindung aus Mercaptoessigsäure, Cystein oder Thiomilchsäure oder deren Salzen ausgewählt ist.

16. Mittel nach einem der Ansprüche 1 bis 15, wobei die Haarkeratin reduzierende Verbindung in dem Wellenformungsmittel in einer Menge von 2 bis 15 Gewichtsprozent enthalten ist.

17. Mittel nach einem der Ansprüche 1 bis 16, wobei das Wellenformungsmittel das Disulfid einer Haarkeratin reduzierenden Verbindung enthält.

18. Mittel nach Anspruch 17, wobei das Disulfid der Haarkeratin reduzierenden Verbindung Dithioglycolat ist.

19. Mittel nach einem der Ansprüche 17 oder 18, wobei das Disulfid der Haarkeratin reduzierenden Verbindung in einer Menge von 2 bis 20 Gewichtsprozent enthalten ist.

20. Mittel nach einem der Ansprüche 17 bis 19, wobei das Verhältnis der Haarkeratin reduzierenden Verbindung zu dem Disulfid der Haarkeratin reduzierenden Verbindung von 2:1 bis 1:1 beträgt.

21. Verfahren zur permanenten Haarformung, wobei das Haar mit dem Haarkeratin reduzierenden Formungsmittel behandelt wird, bevor und/oder nachdem das Haar in eine gewünschte Form gebracht wurde, und nach einer für permanente Formung ausreichenden Einwirkzeit wird das Haar, falls notwendig, mit Wasser ausgespült, dann bekommt das Haar eine oxidative Nachbehandlung und wird erneut mit Wasser ausgespült, in eine Frisur gebracht und letztlich getrocknet, wobei ein Mittel nach einem der Ansprüche 1 bis 20 als das Haarkeratin reduzierende Formungsmittel verwendet wird.

## Revendications

1. Agent de mise en forme réducteur de kératine des cheveux pour effectuer l'étape de réduction dans une mise en forme permanente des cheveux, comprenant au moins un composé réducteur de kératine des cheveux et un dérivé de cellulose cationique qui est formé à partir d'un éther de cellulose avec de 1000 à 10 000 motifs anhydroglucose qui se répètent, qui (a) est substitué avec 0,0001 à 0,1 mole par motif anhydroglucose d'un substituant de Formule
(I) R¹R²R³R⁴N⁺ (A^{z-}) _{1/z}
dans laquelle,
R¹ et R² indépendamment l'un de l'autre représentent -CH₃ ou -C₂H₅,
R³ représente -CH₂-CH(OH)-CH₂- ou - CH₂CH₂-,
R⁴ représente un résidu alkyle à chaîne linéaire ou ramifié avec 6 à 24 atomes de carbone,
A^{z-} est un anion, choisi parmi chlorure, sulfate, nitrate ou phosphate, et
z signifie 1, 2 ou 3, et
(b) est substitué avec 0,01 à 0,9 mole par motif anhydroglucose d'un substituant de Formule
(II) R⁵R⁶R⁷R⁸N⁺ (A^{z-}) _{1/z}
dans laquelle,
R⁵, R⁶ et R⁷ indépendamment l'un de l'autre représentent -CH₃ ou -C₂H₅,
R⁸ représente -CH₂-CH(OH)-CH₂- ou -CH₂CH₂-,
A^{z-} est un anion, choisi parmi chlorure, sulfate, nitrate ou phosphate, et
z signifie 1, 2 ou 3.

2. Agent selon la revendication 1, dans lequel dans la Formule (I), R¹ et R² représentent -CH₃ et R³ représente -CH₂-CH(OH)-CH₂-.

3. Agent selon l'une ou l'autre des revendications 1 ou 2, dans lequel dans la Formule (I), R⁴ représente un résidu alkyle à chaîne linéaire ou ramifié avec 10 à 16 atomes de carbone.

4. Agent selon l'une quelconque des revendications 1 à 3, dans lequel dans la Formule (II), R⁵, R⁶ et R⁷ représentent -CH₃.

5. Agent selon l'une quelconque des revendications 1 à 4, dans lequel dans la Formule (II), R⁸ représente -CH₂-CH(OH)-CH₂-.

6. Agent selon l'une quelconque des revendications 1 à 5, où ledit agent contient un dérivé de cellulose cationique de formule générale (III), 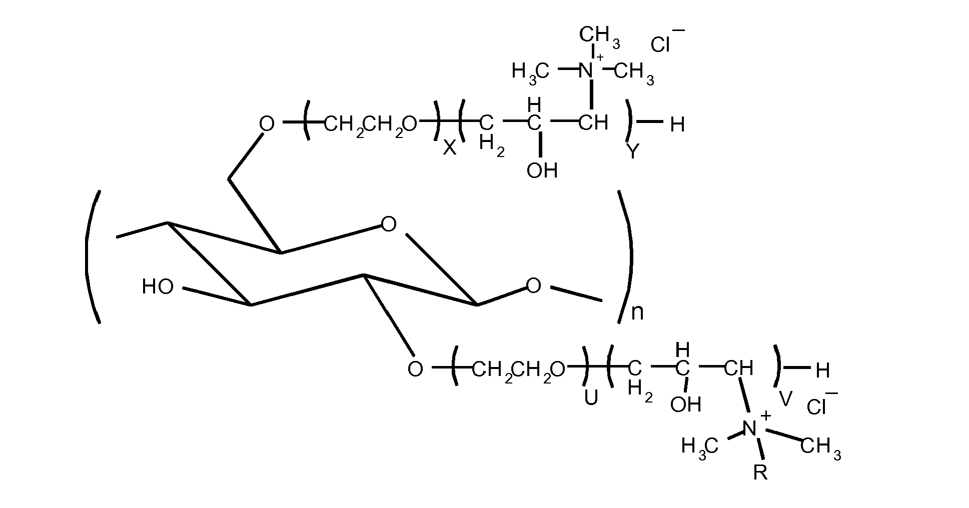 dans laquelle,
X représente un nombre de 0,05 à 0,8,
Y est un nombre de 0,05 à 0,8,
U représente un nombre de 0,0005 à 0,05,
V représente un nombre de 0,0005 à 0,05,
R est un résidu alkyle à chaîne linéaire ou ramifié avec 6 à 18 atomes de carbone et n représente un nombre entier allant de 1000 à 8000.

7. Agent selon la revendication 6, dans lequel dans la Formule (III) R = C₁₂ H₂₅.

8. Agent selon la revendication 1, où ledit agent contient un dérivé de cellulose cationique de Formule générale (IV), 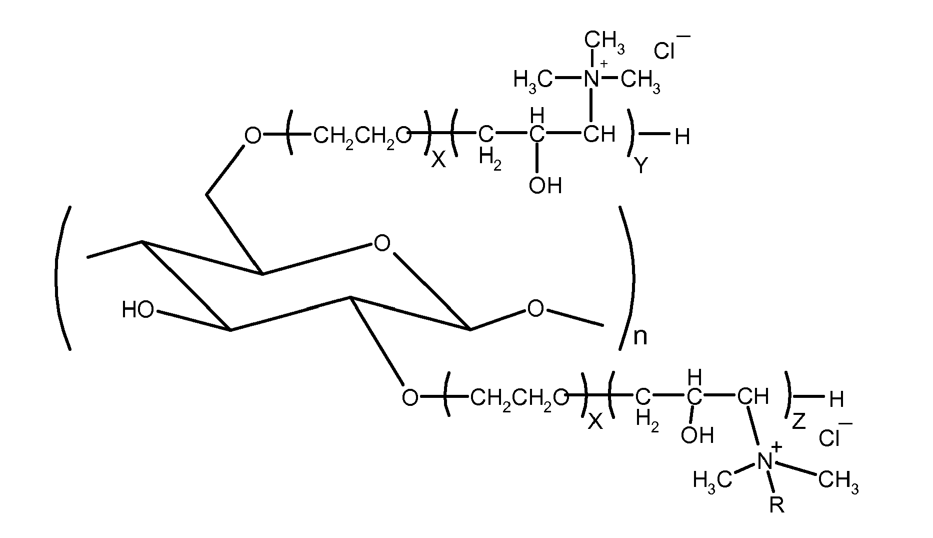 dans laquelle,
X désigne un nombre entier allant de 1 à 10,
Y est un nombre entier allant de 0 à 10,
Z désigne un nombre entier allant de 1 à 10,
R est un groupe alkyle à chaîne linéaire ou ramifié avec 6 à 18 atomes de carbone, et
n désigne un nombre entier allant de 10 à 5000.

9. Agent selon la revendication 8, **caractérisé en ce que**, dans la Formule (IV),
X désigne un nombre entier allant de 1 à 4,
Y désigne un nombre entier allant de 1 à 4,
Z désigne un nombre entier allant de 1 à 4,
R est un groupe alkyle à chaîne linéaire avec 10 à 16 atomes de carbone, et
n désigne un nombre entier allant de 50 à 1000.

10. Agent selon la revendication 8 ou 9, dans lequel dans la Formule (IV) R = C₁₂H₂₅.

11. Agent selon l'une quelconque des revendications 1 à 10, dans lequel une solution aqueuse à 1 % du dérivé de cellulose cationique présente une viscosité de 1500 à 3000 mPa · sec à 25 degrés Celsius (77 °F), mesurée avec un viscosimètre rotatif Haake Modèle VT 550 à une vitesse de cisaillement de 10,0 par seconde.

12. Agent selon l'une quelconque des revendications 1 à 11, dans lequel le dérivé de cellulose cationique est contenu en une quantité allant de 0,1 à 5 pour cent en poids.

13. Agent selon l'une quelconque des revendications 1 à 12, où ledit agent contient en outre au moins un polymère cationique qui est choisi parmi le poly(méthacrylate de diméthylaminoéthyle) (rendu quaternaire jusqu'à 75 % avec du sulfate de diméthyle ou jusqu'à 100 % avec du chlorure de méthyle ou du bromure de méthyle), le poly(chlorure de diallyldiméthylammonium), des copolymères chlorure de diallyldiméthylammonium/hydroxyéthylcellulose, un homopolymère méthosulfate de bêta-méthacryloxyéthyltriméthylammonium, des copolymères méthosulfate de bêta-méthacryloxyéthyltriméthylammonium/acrylamide, des copolymères chlorure de bêta-méthacrylyloxyéthyltriméthylammonium/méthacrylamide, des copolymères d'acrylamide et de chlorure de diméthyldiallylammonium, un copolymère vinylpyrrolidone/méthacrylate de diméthylaminoéthyle, des copolymères méthosulfate de bêta-méthacryloxyéthyltriméthylammonium/vinylpyrrolidone, un copolymère chlorure de méthylvinylimidazolium/vinylpyrrolidone, des copolymères chlorure de diallyldiméthylammonium/acrylamide, des copolymères N-vinylpyrrolidone/chlorure de méthacrylamidopropyldiméthylammonium, des dérivés de chitosan cationique, des dérivés de cellulose cationique différents des dérivés de cellulose cationique selon la revendication 1 et des dérivés de gomme guar cationique.

14. Agent selon l'une quelconque des revendications 1 à 13, dans lequel la quantité totale de polymère cationique est de 0,2 à 5 pour cent en poids.

15. Agent selon l'une quelconque des revendications 1 à 14, dans lequel le composé réducteur de kératine des cheveux est choisi parmi l'acide mercaptoacétique, la cystéine ou l'acide thiolactique, ou leurs sels.

16. Agent selon l'une quelconque des revendications 1 à 15, dans lequel le composé réducteur de kératine des cheveux dans l'agent d'ondulation est contenu en une quantité allant de 2 à 15 pour cent en poids.

17. Agent selon l'une quelconque des revendications 1 à 16, dans lequel l'agent d'ondulation contient le disulfure d'un composé réducteur de kératine des cheveux.

18. Agent selon la revendication 17, dans lequel le disulfure du composé réducteur de kératine des cheveux est du dithioglycolate.

19. Agent selon l'une ou l'autre des revendications 17 ou 18, dans lequel le disulfure du composé réducteur de kératine des cheveux est contenu en une quantité allant de 2 à 20 pour cent en poids.

20. Agent selon l'une quelconque des revendications 17 à 19, dans lequel le rapport du composé réducteur de kératine des cheveux sur le disulfure du composé réducteur de kératine des cheveux va de 2:1 à 1:1.

21. Procédé pour la mise en forme permanente des cheveux, dans lequel les cheveux sont traités avec un agent de mise en forme réducteur de kératine des cheveux avant et/ou après que les cheveux ont été mis dans une forme souhaitée, et après une période d'action suffisante pour la mise en forme permanente, les cheveux sont rincés avec de l'eau si nécessaire, puis les cheveux reçoivent un posttraitement oxydant, et sont rincés de nouveau avec de l'eau, sont coiffés selon une coupe et enfin séchés, dans lequel un agent selon l'une quelconque des revendications 1 à 20 est utilisé en tant que l'agent de mise en forme réducteur de kératine des cheveux.
